# EUROPEAN PATENT APPLICATION

(11) **EP 1 439 387 A1**
(43) Date of publication of application: **21.07.2004**
(21) Application number: 02714230.6
(22) Date of filing: 20.03.2002
(51) Int. Cl.: G01N 27/27

(54) **INSTRUMENT AND METHOD FOR THE ANALYSIS, IDENTIFICATION AND QUANTIFICATION OF GASES OR LIQUIDS**

(30) Priority: 26.03.2001 ES 200100742
(71) Applicant: Universidad de Barcelona, 08028 Barcelona (ES)
(72) Inventor: PERERA LLUNA, Alexandre, E-08800 Vilanova I La Geltrú (ES); SUNDIC, Teodor, E-08012 Barcelona (ES); PARDO MARTINEZ, Antonio, E-08970 Sant Joan Despi (ES); MARCO COLAS, Santiago, E-08750 Molins De Rei (ES); GUTIERREZ OSUNA, Ricardo, E-08028 Barcelona (ES)
(74) Representative: Torner Lasalle, Elisabet
(86) International application number: PCT/ES2002/000134
(87) International publication number: WO 2002/077631

(57) **Abstract**

The instrument includes a housing; at least one sensor module including an analysis chamber (5) with several chemical sensors (14); at least two inlet pipes (1a, 1b, 1c) and associated means (3a, 3b, 3c, 7) to perform selective circulation of gases or liquids through this chamber (5); a unit (17) for the processing of the data collected by these sensors (14) and control of the operative one of these fluidic means (3a, 3b, 3c, 7); and peripheral modules (19, 20, 21) associated to the applied processing unit (17), jointly with this unit, to a control of the operation of the sensors (14) providing a sensor excitation adapted for each specific application based on the characteristics of composition of the sample to analyse.

## Description

### Field of the invention

The present invention concerns to an instrument for carrying out an analysis of a gas or liquid substance, and for identifying and quantifying their components. The invention also concerns to a method wherein one or more of such instruments are used, to a method for optimising its operation, and to a system constructed using a series of such instruments conveniently interconnected.

### Antecedents of the invention

Nowadays several systems for analysis, identification and quantification of gases or liquids are known, which are available commercially being offered by diverse companies. The most habitual applications are quality control in the food industry, the medical diagnosis, the environmental control and the security, among others.

The systems for gas analysis, identification and quantification try to cover the market for devices that imitate the human olfactory perception, reason why commonly are denominated "electronic noses". Throughout this memory said brief expression "electronic noses" will often be used to make reference to such systems.

The possibility of measuring aromas by electronic means is a great challenge. In the industrial scope, and concrete in the food industry and cosmetic industry, the evaluation of the aromas is made by means of human panels, usually complemented with analytical instrumentation techniques like gas chromatography and mass spectroscopy (GC/MS). In spite of their evident relation with the human evaluation of the scent, these methods are very tedious, expensive and seldom used in real time or field tests. The possibility of being able to use systems based on gas sensors working in real time allowing the evaluation of the aroma in specific points, would open multitude of new applications. In addition it would allow solving other problems associated to the use of human panels: individual variability, adaptation (the humans we become less sensible after a prolonged exhibition), fatigue, mental state, subjectivity and impossibility to evaluate toxic compounds.

Although the gas sensors have been known for decades, the concept of electronic nose goes a step further on. Its base is a matrix of chemical sensors that responds to a variety of compounds in different degree. The signals of this matrix are processed by means of pattern recognition algorithms to extract the relevant information. The electronic noses are usually sold in the developed countries, such as the United States, Europe and Japan, at relatively high prices and their use is normally restricted to the laboratory.

In spite of their colloquial name, the electronic noses are still far of their biological model. At heart of the nasal cavity of the human sense of smell, around 10⁸ receivers of 10³ different types are lodged. Each type of receiver is characterised by a particular pattern of sensitivities. The signals of these receivers are pre-processed in the olfactory bulb, where the number of 25000 glomerules approximately is concentrated. Later, 10⁸ neurons are in charge of the olfactory signals final processing in the cortex. However, in commercial electronic noses, the matrix of sensors consists only on between 6 to 20 sensors with the associated signal processing techniques that are rather modest compared to the human olfactory system.

In the electronic noses, the discrimination power of different scents depends in a great extent on the diversity of chemical information that the sensors can provide. Various types of sensors are used for this purpose, permitting certain diversification of its sensitivity properties. In Table 1 the most common gas sensor technologies are listed specifying the type, principle of operation, availability and how its profile of sensitivity can be modified.

**Table 1**

| **Type** | **Principle** | **Availability** | **Method of diversification** |
|---|---|---|---|
| Surface Acoustic waves (SAW) | Change of mass | Restricted | Polymer Type |
| MOSFET | Change of voltage threshold | Very Restricted | Catalytic Metal Thickness Temperature |
| Quartz Microbalance (QMB) | Resonant detection of absorbed mass | Restricted | Polymer Type |
| Conductive polymer (CP) | Change conductivity in volume | Very Restricted | Polymer Type |
| Calorimetrics | Heat of reaction | Extensive | Catalytic Temperature |
| Optics | IR Absorption | Extensive for some gases | Wavelength Selection Filter |
| Electrochemical | REDOX Reaction | Extensive | Catalytic Voltage |
| Metal Oxide (MOX) | Change resistivity by superficial reaction | Extensive | Doping Material Morphology Work Temperature |

Each type of sensor has its advantages and disadvantages. For example, the tin oxide sensors do not detect CO₂; however it can be detected optically by its absorption in the infrared. The conductive polymer sensors are very sensible to the humidity. The calorimetric sensors detect only inflammable gases. Sensors QMB and SAW are especially sensible to big molecules and posses more linear response than the MOX. The lack of linearity of MOX sensors has important consequences because it increases the dispersion of the patterns.

Majority of electronic noses actually present in the market incorporate a unique type of sensor in order to optimize the design of the instrument. In some occasions several sensor types can combine in the same instrument. For example, the Lennartz Electronics Company offers the MOSES modular system that allows incorporating a module QMB or a module MOX. Other example is Applied Sensors that combines in one instrument MOSFET and MOX sensors. Other types of sensors, such as electrochemical, calorimetric, IR, are not used in commercial devices although they are present in the literature. Recently, in the instrumental scope, the use of TENAX pre-concentrator has raised a lot of interest, although only one commercial model incorporates it [W. Muenchmeyer, To Walte, G. Matz, Improving Electronic Noses using the Trap and thermal desorption unit., in 6th Int. Symp. on Olfaction and Electronic Noses, Tuebingen, September 1999, pp. 7-10]. This type of pre-concentrator not only allows increasing the sensitivity of the instrument but also allows a greater flexibility for the optimization of the operation because the temperature of desorption can be used as an operating parameter. The use of chemical filters at the sensorial chamber input also posses certain advantages and is very common in the commercial devices.

It is possible to emphasize that only recently the necessary technology for the manufacture of resistive sensors based on metallic oxides on micromechanized silicon substrate has been developed. This last point is important due the sensor response dependency on temperature that permits fast temperature modulation. Performing this modulation more information can be extracted.

The present invention proposes the use of hybrid sensorial matrices that combine different technology devices in order to maximise the chemical information that arrives at the instrument processor.

Recently, similar concepts for the analysis of liquids have been adapted and the term of "electronic tongue" or "taste sensor" has been proposed to make reference to devices applied to such analyses. This electronic tongue is based on three-electrode configuration (includes certain number of metallic working electrodes together with a reference electrode and a counter electrode). These electrodes are controlled by means of a potenciostat. The response is the current level that corresponds to a certain potential between the working and the reference electrode. These metallic electrodes are not selective; their response depend on their chemical composition and the working voltage [F. Winquist, P. Wide, I. Lundström, An electronic tongue based on voltammetry., Anal. Chim. Act. 357 (1997) 21-31]. By means of these electrode arrays the analysis of samples like fruit juices, mineral water or milk can be performed. Moreover, they have been used for monitoring of the bacterial growth in food samples in liquid state.

The signal processing of the data collected by the sensors deserves a special attention. In this field exist a great variety of techniques that can be implemented. Among all of them, classic chemometric techniques, featuring Principal Component Analysis (PCA) and Linear Discriminate Analysis (LDA), are dominating ones [ M. Otto, "Chemometrics", Wiley-VCH, 1999 ].

Nevertheless, in the last decade, the artificial neural networks based on multilayer perceptrons have been successful in this field [ J.D. Kim, H.G. Byun, K.C. Persand, "Application of to multilayer perceptron based on the Levenberg-Mardquart algorithm to odour pattern classification and concencration estimation using odour sensing system", 7th Int. Symp. on Olfaction and Electronic Nose, Brighton 2000, pp. 115-120, IOP pub ]. The special contribution of the developments based on neuronal network algorithms is that they have the capability, if they are designed correctly, to solve problems that intrinsically contain a certain degree of not-linearity. This case is not unusual given the type of response offered by some of above described sensors.

In addition, another kind of advanced algorithms that could be implemented in this field have been described, as for example:
- RBF neural networks (Radial Basis Functions) [B. Walczak, D.L. Massart, Local Modelling with Radial Basis Functions. "Chemometrics and Intelligent Laboratory Systems" 50, 179-198 (2000) ], [ T. Sundic, S. Marco, A. Perera, A. Pardo, J. Samitier, P. Wide. "Potato creams recognition from electronic nose and tongue signal: feature extraction/selection Neural and RBF Networks classifiers", in NEUREL 2000, Belgrade, 2000, pp. 69-74 ]; are a particular type of neural networks which use gaussian transfer function, instead of the typical ones used in multilayer perceptrons (step, linear or sigmoid transfer functions).
- Fuzzy inference algorithms [ P.Y. Glorennec, "Algorithmes d'apprentissage pour systèmes d.inférence floue", Hermes. Paris 1999, ], [ T. Sundic, A. Perera, S. Marco, A. Pardo, A. Ortega, J. Samitier. ''Fuzzy Logic processing in combined coal and methane domestic gas alarm", in 7th Int. Symp. on Olfaction and Electronic Noses, Brighton, Julio 2000, pp. 165-170. IOP Pub ], is a different approach to the signal processing and pattern classification problems in which it is possible to systematise diffuse concepts in relation, for example, to the property degree of an object to a certain class.
- Neuro-fuzzy systems, as a combination of aspect s related to the neuronal networks and the fuzzy inference (J.S.R. Jang, "ANFIS: Adaptative-Network-Based Fuzzy Interference Systems", IEE Transactions on Systems, Man and Cybernetics, Vol. 23, No. 3, pp. 665-685, May 1993].
- Methods for mixture models estimation based on EM algorithms [ M.A.T. Figuereido, A.K. Jain, "Unsupervised Selection and Estimation of Finite Mixture Models", 15th International Conference, on Pattern Recognition, Barcelona 2000, pp. 87-90, vol. 2. IEEE Press ].
- Feature selection algorithms (SFS, SBS, SFFS), that allow size reduction of the problem on one side, and the processing algorithms performance by means of a systematic selection of the suitable parameters on the other [A. Pardo, S. Marca, C. Calaza, A. Ortega, A. Perera, T. Sundic, J. Samitier, "Methods for sensors selection in pattern recognition", in 7th Int. Symp. on Olfaction and Electronic Noses, Brighton, Julio 2000, pp. 83-86. Iop Pub], [T. Sundic, S. Marco, To Perera, Pardo, J. Samitier, P. Wide. "Potato creams recognition from electronic nose and tongue signal: feature extraction/selection Neural and RBF Networks classifiers", in NEUREL 2000, Belgrade, 2000, pp. 69-74]
- Genetic algorithms, based on methods inspired by the biology and particularly by the survival Darwinian rules [A. Pardo, S. Marco, C, Calaza, A. Ortega, A. Perera, T. Sundic, J. Samitier, ''Methods for sensors selection in pattern recognition", in 7th Int. Symp. on Olfaction and Electronic Noses, Brighton, Julio 2000, pp. 83-86. Iop Pub].

The drift compensation methods are also of extreme interest [S. Marco, A Pardo, A Ortega, J. Samitier, ''Gas identification with tin oxides sensor array and self organising maps: Sensorial Adaptive correction of drifts", IEEE Transactions on Instrumentation and Measurements Vol 47 N° 1 pp 316-321 (1998)], as also the transference of calibration between different units [C. Distante, T. Artursson, P. Siciliano, M. Holmberg, I. Lundstrom, "Odour identification to under drift effect", in 7th Int. Symp. on Olfaction and Electronic Noses, Brighton, Julio 2000, pp. 89-96. Iop Pub].

As it has already been mentioned, the electronic nose has a wide field of applications and it has been used to deal with many different kinds of problems. Some of the more significant applications are briefly mentioned in continuation.

In the food industry it has been used for tasks such as freshness control, quality control and selection of base products, control of twin-jets, monitoring of accidental contamination, labelling. Examples in this area include the evaluation of the olive oil aroma [M. Pardo, G. Sberveglieri, S. Gardini, And Dalcanale, "Sequential Classification of 14 Olive Oil Types by an Electronic Nose", in 6th Int. Symp. on Olfaction and Electronic Noses, Tuebingen. September 1999, pp. 253-259 ], the determination of the fruit maturity [J. Brezmes, E. Llobet, X. Vilanova, G. Saiz, X. Correig, "Non-destructive fruit ripeness monitorization using tin dioxide gas sensors and pattern recognition algorithms", 6th Int. Symp. on Olfaction and Electronic Noses, Tuebingen, September 1999, pp. 259-262 ], cheeses aroma [C.N, Raynaud, C.M. Delahunty, "Differentiation of Cheddar Cheeses Made with two different Cheese Starter cultures using an Electronic Nose ace compared to sensory analysis and cheese clay scores", 6th Int. Symp. on Olfaction and Electronic Noses, Tuebingen, September 1999, pp. 282-285] sauce recognition [ T. Sundic, S. Marco, A. Perera, A. Pardo, J. Samitier, P. Wide. "Potato creams recognition from electronic nose and tongue signal: feature extraction/selection Neural and RBF Networks classifiers". in NEUREL 2000, Belgrade, 2000, pp. 69-74 ], etc.

In biotechnology several applications related to the bacterial identification and the control of their growth have been developed. The techniques of conventional detection require baseline concentrations of 10³ colonies per ml, but faster detection would be necessary. Moreover, the main problem of the conventional techniques is its incapability to distinguish the bacterium state: therefore it cannot discriminate between dead or alive bacterium. The real time analysis of the bacterial growth has great relevance as it can be used for the control the nutrient contribution, the relative growth of different species or its reaction to antibiotics. Gardner et al. [ J.W. Gardner, C.S. Dow, H.W. Shin, G. Searle, MJ. Chappel, "Dynamical Signal Processing for Techniques bioprocess monitoring", 6th Int. Symp. on Olfaction and Electronic Noses, Tuebingen, September 1999, pp. 331-335 ] uses an electronic nose in combination with measures of electrical impedance to control the growth of Staphylococcus. However, Haugen et al. [J.E. Haugen, And Witso, K. Naterstad, H. Nissen, S. Bredholt, ''Discrimination of lactic acid bacterium by using to hybrid sensorial gas Array: chemotaxonomic and genetic correlations", 6th Int.. Symp. on Olfaction and Electronic Noses, Tuebingen, September 1999, pp. 338-340 ] applies the electronic nose to the identification of different strains from lactic acid bacteria.

In the medical field, the electronic nose can be used to analyse the breath, swat, urine, etc. and to help a premature diagnosis. For example, the interest of this type of instruments in the processing of the halitosis can also be mentioned. This problem normally appears in the dentist consultations and it is estimated that a 50% of the population can suffer it in some moment of their life. Unfortunately, social consequences are not the only ones, but often halitosis reflects other pathologies like e.g. lung or pharynx cancer, Sjogren syndrome, diabetes, gastric problems, renal and hepatic failures, etc. The interest in the clinical use of an electronic nose in this context has been reviewed in [S. Nachnani, "Oral malodour", in 7th Int. Symp on Olfaction and Electronic Noses, Brighton, Julio 2000, pp. 3-12. IOP Pub]. Moreover, electronic nose has been used to analyse the scent produced by medicines [ SS Schulman, B.G. Kaman, H.T. Angle, "Analysis of medication off-odors using an electronic nose", Chemical Senses, 22, 119-128 (1997) ].

In the security field the emphasis has been put to premature fire detection and the identification of burning substances. It would permit the important decrease of false alarms on one hand, and the realisation of specific measurement depending on the fire types on the other. The last progresses at this respect can be revised in [S.L. Rose-Pehrsson, R.E. Shatter, D.T. Gottuk, "Probabilistic Neural for Network Early Fire Detection using an Electronic Nose", in 6th Int. Symp. on Olfaction and Electronic Noses, Tuebingen, September 1999, pp. 195-198].

In the environmental field there are to be emphasised applications referring to the control of malodorous emissions generated by dumps, purifying plants, and chemical industries in general. Furthermore, the availability of a network of such instruments enables the preparation of maps showing the distribution of contaminants in the environment, see [Alex Perera, Toni Pardo, Teodor Sundic, Ricardo Gutierrez-Osuna, Santiago Marco, Jacques Nicolas, "Ip Nose: Electronic Nose for Remote Bad Odour Monitoring System in Landfill Sites", Computer Science Department Wright State University, Daytona Ohio, USA, Sistemes d'instrumentació I comm., Departament d'Electrònica, Universitat de Barcelona, Barcelona, Spain, y Pondation Universitaire Luxembourgeoise, Environmental Monitoring Belgium].
There exist several patents concerning instruments and/or methods of nose and/or electronic tongue. For example, patent US-A-6085576, of Cyrano Sciencies, Inc., which describes a handheld device for vapour sensing, lightweight and manageable that can be adapted for use in sensing the presence and concentration of a wide variety of specific vapours. The device includes a sensor module that incorporates a variety of sensors located inside a sample analysis chamber, through which the sample is directed to pass. The sensors provide a combination of electrical signals. The sensors can take the form of chemical resistors having resistances that vary according to the identity and concentration of the adjacent vapour. These chemically sensitive resistors can be connected in series with a reference resistor, between a reference voltage and ground, such than an analog signal is established for each chemically sensitive resistor. The resulting analog signals are supplied to an A/D converter to produce corresponding digital signals, which are appropriately analysed for vapour identification.

The main disadvantage that presents the Cyrano device is that it does not anticipate a modulation of the sensor operation parameters to obtain different responses from each one. For this reason, it needs a great variety of different types of sensors to detect a variety of different vapours or gases, or it must install a single type of sensors to detect a certain specific vapour.

On the other hand, patent US-A-4818348 presents a method and an apparatus for identifying and quantifying simple and complex chemical products, either gases or liquids or solids. The instrument includes an array of small sensors that, upon exposure to the unknown material, form a pattern of electrical responses that are compared, by means of a microprocessor, with one or more patterns coming from a source of previously formed response patterns characteristic of various materials to thereby identify the material on a display. The number of responses may be increased changing the operating voltage, temperature or other conditions associated with one or more sensors to provide a plurality of responses from each one of the sensors. Depending on the type of sensor array therein, the instrument way analyse gases or liquids, and includes means for changing a gas, liquid or solid in a fluid material.

Although in the Stteter above patent, the possibility of obtaining different responses modulating diverse sensor operating parameters is considered, at no moment describes a procedure to repeatedly vary ranks of values of one or several of these operation parameters based on the result to compare the obtained response patterns by means of a previous rank of values with the stored characteristic patterns, until reaching a convergence criterion, with the purpose of obtaining a better result in the detection and quantification of a specific substance.

Also, in none of the two mentioned patents, Cyrano and Stetter, the possibility of connecting a system using several of such instruments to a computer network to carry out either a control or a programming or reprogramming of the system from a remote place is mentioned. It's to say, in the prior art reflected by those two cited patents, an individual operation of the instruments being either in situ or eventually remote controlled is intended, but interconnection of several such instruments to perform a distributed analysis of data generated by said instrument set or network is not envisaged.

### Summary of the invention

The present invention provides a device and a method for the analysis, identification and quantification of gases or liquids, commonly denominated electronic nose (for gases) or electronic tongue (for liquids), based essentially on the integration of a small computer embedded within an instrument. This provides a large memory capacity and data processing, enough to store and to process all the information relative to the operation of the equipment that allows:
- The use of an arbitrary modulation of the sensors excitation temperature, tin oxide sensors for example, in a compact, portable equipment;
- Storage of large amounts of data;
- An advanced signal processing; and
- A remote connectivity through a computer science network.

The election of embedded technology provides interesting benefits: the existence of an abstraction layer for acquisition and control through an operating system, the high level programming of the signal processing algorithms, great capacity of storage through hard discs, hardware prepared for the Ethernet connectivity, serial ports, interfaces for all type of screens, etc, Options for the operating system are embedded Microsoft NT, Windows CE or UNIX clone with open sources. Last one is preferred because the accessibility to the sources that allows an adjustment of operating system to the hardware size needs, although it would also be feasible to work with others systems.

According to a preferred exemplary embodiment, the instrument may include up to three sensor modules, including four tin oxide sensors in each module, a temperature sensor, the conditioning circuitry for sensor signals and the electronics for the sensor excitation.

The sensors are mounted on an acquisition board covered by a stainless steel chamber. The design of the electronics allows the incorporation of any type of commercial sensors through configuration jumpers.

The injection and sample delivery system can be extended and both, software and hardware are prepared for the incorporation of additional components, such as for example PWM (Pulse Wave Modulation) temperature controller or Tenax type odor concentrator. The embedded system takes care of sensor signal acquisition and of generating the excitation waveform, whereas another peripheral module is responsible for the subsystem flow control (pump and valves). The enclosure of a Linux type operating system allows the use of the typical characteristics like multitasking, shared libraries, TCP/IP connectivity or possibility of multi-user function. The whole system has been formed using text files. These files are accessible to the user, in such a way that the user can freely access to the definition of cycles number, cycle times, status of the nose in each phase of measurement, as also sampling times and arbitrary waveform, etc.

Specifically, the instrument for the analysis, identification and quantification of gases or liquids according to the present invention includes:
a housing;
at least one sensor module incorporating an analysis chamber that lodges at least one chemical sensor;
at least two inlet ports and associated means for selectively introducing in a controlled way two or more gas or liquid samples to the analysis chamber, and for circulating these samples therethrough, constituting a programmable flow subsystem;
a unit for processing the data collected by said at least one sensor and for the operation of the means to introduce and to make circulate the samples of gases or liquids; and
peripheral modules associated to said processing unit, applied jointly therewith to an active control of the operation of said sensor, that is at least one, providing an excitation waveform for this sensor adapted for each specific application, based on the characteristics of the sample/samples composition to analyse.

In a variant apt for the gaseous data sampling, the instrument sensor module includes a set of four or more metallic oxide sensors and a temperature sensor associated to this metallic oxide sensors set, which is located inside the analysis chamber.

In a variant appropriate for the data extraction from liquid samples, the instrument sensor module includes one or more non-selective metallic work electrodes, a reference electrode and a counter electrode, altogether being located inside the analysis chamber, being governed by a potenciostat.

Thanks to the developed design concept, the device is, according to an embodiment, a portable chemical analysing system for vapours and it can be applied to many and very diverse applications. Among other applications, the following ones may be highlighted: the food industry, biomedical characterisation, air quality control, either in closed space or in opened space, etc. Also it is possible to use it for acquire a large amounts of data in field, for a later analysis in laboratories or a real time processing. All the transferable data are stored in compatible files with Matlab or OCTAVE to allow an easy later processing outside the instrument if it is necessary. The modularity, software compatibility and portability allow the user to verify the algorithms and to collect data when the vapour or odor cannot be taken to the laboratory. Users are allowed to program their signal processing code, to compile it using a standard gcc and to include it in the nose-processing engine. The whole system may work in a remote mode through TCP/IP under the client/server structure, so Internet can make the distributed or remote instrumentation and measures. That can be useful when the processes, like for example quality control, have to be carried out in several points of a production line. With the remote operation mode, the data result of the distributed process can be centralised in just one computer unit. In addition, long time test and stability test can be automated easily using the standard planing commands of UNIX (for example *cron* or *at*) and the user can receive an electronic mail of the nose, reporting a failure or degradation of the measurement.

### Brief description of the drawings

Next, preferred exemplary embodiments of the present invention are described with reference to the attached drawings, in which:
Fig. 1 is a general fluid circuit scheme and means associated to it used for directing of gaseous sample through a sample analysis chamber in a controlled way, constituting a fluidic subsystem in an exemplary embodiment of the invention applied to gaseous samples;
Fig. 2 shows a schematic detail of a lateral section view of the sample analysis chamber of Fig. 1;
Fig. 3 shows a scheme of a control system that includes a processing unit and associated peripheral modules for the control of the fluidic subsystem of Fig. 1, the excitation of the sensors located in the chamber of Fig. 2 and the data processing from sensors responses;
Fig. 4 shows a scheme of one of the peripheral modules of Fig. 3 dedicated to the excitation of the sensors;
Fig. 5 shows a scheme that illustrates a variant of implementation of the invention in which several sample chambers of analysis are connected, in the fluidic sense, in series;
Fig. 6 shows a scheme that illustrates a distributed network of sensor arrays; Fig. 7 shows a graph that illustrates the sensor responses excited with a sawtooth wave when sensors are exposed to vinegar;
Fig. 7 shows a graph that illustrates the sensor responses excited with a sawtooth wave when sensors are exposed to vinegar;
Fig. 8 shows a graph that illustrates the sensor responses excited with a pulse wave when sensors are exposed to vinegar;
Fig. 9 shows a graph that illustrates an olfactory map for coffee, wood of cedar, tobacco and air with sensor pulse modulation; and
Fig. 10 is a diagrammatic representation of a system for analysis, identification and quantification of gases and liquids comprised of a series of instruments like those proposed by the invention, linked in a logic form.

### Detailed description of preferred exemplary embodiments of the invention

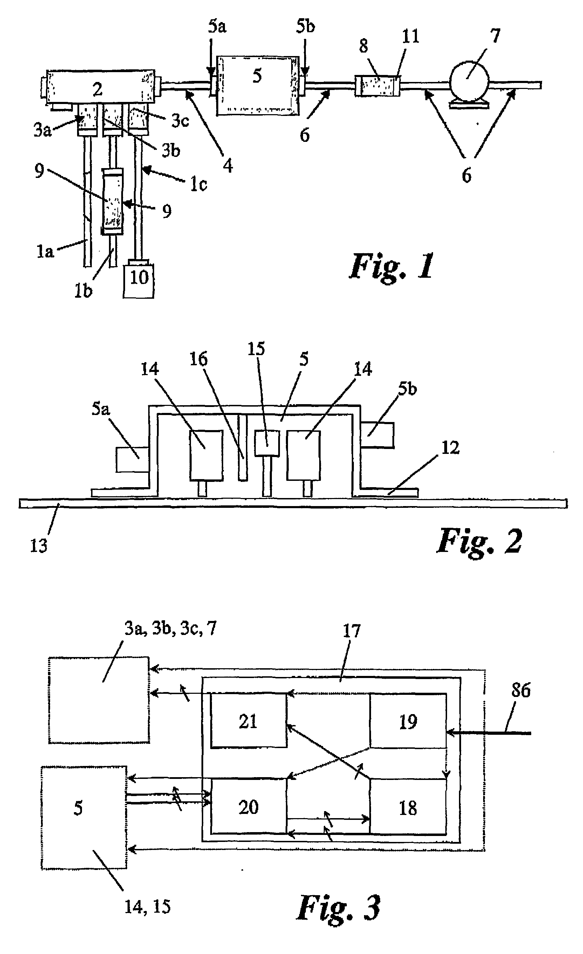

Reference is made first to Fig. 1. This figure shows a fluidic subsystem as a part of the instrument according to the present invention and that includes three inlet ports 1a, 1b, 1c (although they also could be at least two or more than three) connected to a manifold/selector 2 by means of respective input valves 3a, 3b, 3c, for example, two channel valves. A conduit 4 communicates a single exit of said manifold/selector 2 with an entrance 5a of a sample analysis chamber 5. An outlet pipe 6 is connected to an exit 5b of the chamber 5 whose outlet pipe is connected to a pump 7. In said outlet pipe 6, between the chamber 5 and pump 7, a retention valve 8 is allocated. A control module 17 (that is explained below, in relation to Fig. 3) controls the pump action 7 according to the input valves 3a, 3b, 3c to make sequentially circulate at least two different flowed samples through the sample analysis chamber 5. In fact it is possible to make circulate sequentially so many different flowed samples, as inlet pipes are included in the instrument, that is, up to three samples according to the exposed example. Commonly, one of the samples is a known composition fluid that is used as reference in a first cycle of analysis followed by a sample cycle of analysis whose composition is desired to identify or to quantify. It is evident that if, for some specific application, the analysis of a sample coming from a mixture of two different fluids would be needed, this mixture could be obtained operating the input valves 3a, 3b, 3c to simultaneously admit two different fluids through manifold/selector 2 after the reference cycle. In one of the inlet pipe 1b an input filter 9 can be mounted, and another inlet pipe 1c can come from a 10 bubbler or another accessory.

The fluidic system of Fig. 1 is small and can be mounted on the same printed circuit board that incorporates the control electronic module 17, All the assembly is allocated in a housing (not shown) making up an instrument susceptible to be transported and handled manually for works in field. According to a preferred way of implementation, the inlet pipes 1a, 1b, 1c, 4 and first portion of outlet pipe 6 are PVDF tubes and the rest of outlet pipe 6 is polyurethane tubes. The tubes connections with the valves 3a, 3b, 3c, 8, chamber 5, filter 9 and bubbler 10 are bridles of fast assembly 11, such as pressure fit bridles.

With reference to Fig. 2, the chamber 5 includes a small housing of a mechanical and chemical resistant material, as it is the stainless steel, provided with an open face surrounded by a perimetric flange 12, whose housing leans by this open face against the substratum of the sensors conditioning and exciting printed circuit board 13 incorporated in the control electronics 17, to whose substratum is adhered through this flange 12 forming the hermetic close chamber 5 except by the mention inlet and outlet 5a, 5b connected to the corresponding pipes 4, 6 by means of the mention bridle 11. Inside chamber 5 several sensors 14 sensible to gases and a sensor of temperature 15 are allocated. Sensors 14 can be anyone of the above-mentioned types, although for a majority of applications with gaseous samples the metallic oxide sensors are preferred, whereas for liquid samples one or more non-selective metallic work electrodes, a reference electrode and a counter electrode are preferred. In fact, and by virtue of the characteristic excitation control by means of which sensors work, a single gas sensor 14 (or working electrode for liquids) would be enough, although there is no special limit in the number. In the illustrated example, chamber 5 includes a partition 16 that divides its inner space in two compartments, in each one of which there are two gas sensors 14. Partition 16 does not fit completely in the walls of chamber 5 so that those mentioned compartments are in fluidic communication. In one of the compartments, the temperature sensor 15 accompanies the gas sensors 14.

Fig. 3 shows schematically the control module 17 that governs means of selection and circulation of the fluid, that is, valves 3a, 3b, 3c and pump 7, as well as the excitation of the sensors 14, and performs the processing of data acquired by sensors.

This control module 17 is fed by a line 86 coming from a suitable power supply and includes a processing unit 18 in the form of an embedded computer, connected to the previously mentioned peripheral which, at least, include a feeding module 19, a sensor excitation and signal acquisition module 20, connected to the gas sensors 14 and temperature sensor 15 inside the chamber 5, and a power module 21 to drive valves 3a, 3b, 3c as well as pump 7.

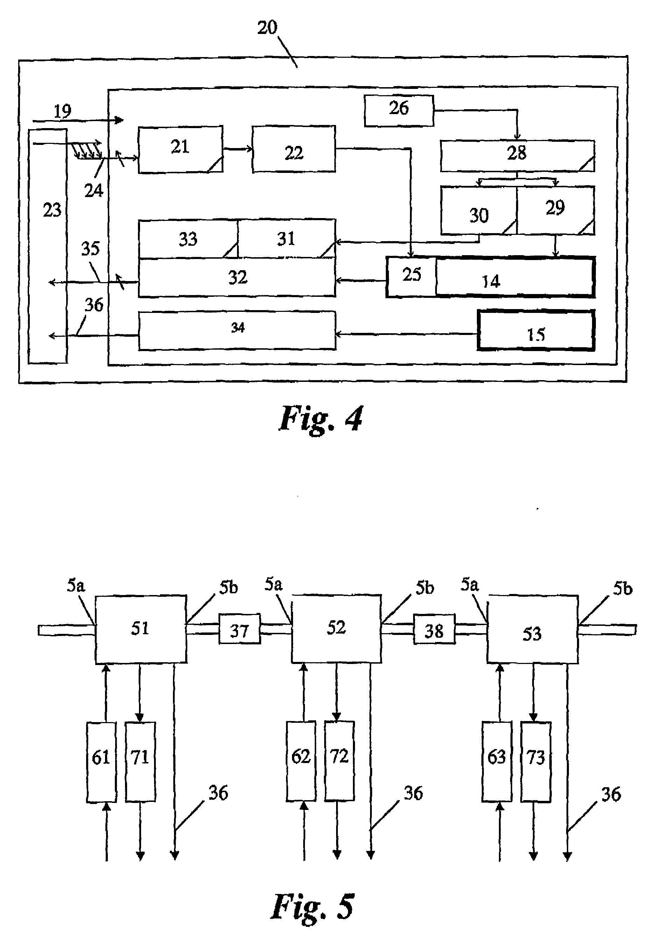

Fig. 4 shows in more detail said sensor excitation and signal acquisition module 20, which is fed from said feeding module 19 and includes a microprocessor 23 that controls the heating of associated heaters 25 of the gas sensors 14 in a modulated form according to predefined wave patterns set. For it, microprocessor 23 sends signals 24 to an adjustable voltage limiter 21 and a buffer 22 performs signal 24 conditioning. On the other hand, an adjustable feeding 26 sends a current 27 to an adjustable current limiter 28 that feeds adjustable load resistance 29 which determine a rank of work feeding of the gas sensors 14, which send a signal of exit 35 towards microprocessor 23 through differential amplifiers 32. Said current limiter 28 also feeds a reference pattern module 30 that sends a signal towards a reference charmels module 31 that along with an adjustable gain 33 calibrate the output sensor signals 14. On the other hand, a temperature sensor 15 produces a signal 36 that is amplified by an amplifier 34 before arriving at microprocessor 23.

Fig. 5 shows a variant embodiment of the instrument of the present invention in which several sample chambers 51, 52, 53 of analysis are connected in series, that is, with the output port 5b of one connected to the input port 5a of the other. Between each two chambers 5 a chemical filter 37 prepared to retire one or more sample components is arranged. Different filters 37, 38 are selected so that they have a capacity of increasing absorption, so that a sample arrives to first chamber 51 where it is analysed by the corresponding sensors, next goes through the first filter 37, which retires one or more sample components, so that the sample arrives at second chamber 52 with some less components, and where is analysed for a second time. The same sample later is filtered in following filter 38, which retires other sample components, and is analysed once again in chamber 53, and so on until finally, after passing through the last chamber, it is expelled through the outlet pipe 6. For each chamber 51, 52, 53, a power circuit 61, 62, 63 for the excitement heating of the sensors and a sensor signal conditioning circuit 71, 72, 73 are arranged.

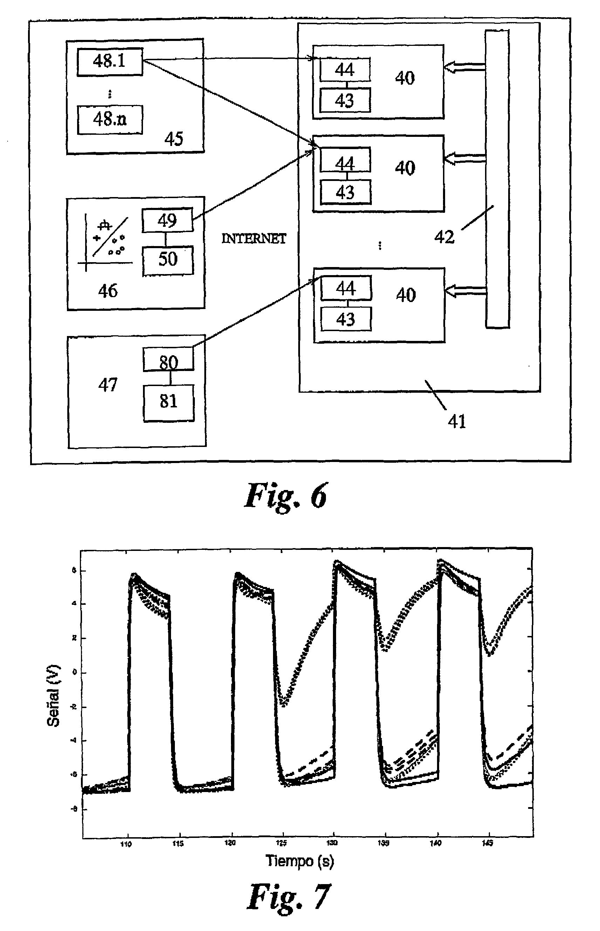

Fig. 6 shows, by way of example, an industrial production plant 41 in which several electronic nose instruments 40 according to the present invention, connected operative with different plant 40 processes 42 are installed. Each instrument 40 includes a user graphical interface 43 and a remote service supplier 44 through as it is connected, through a computer science network, with several remote centres, such as a processes control centre 45, a quality control centre 46 and a research and development centre 47. The processes control centre 45 includes a series of registries 48.1... 48.n in which the data generated by the different sensors from instruments 40 is recorded. The quality control centre 46 includes an user graphical interface 49 through which is possible to see and to analyse received data 50 of instruments 40 and is possible to modify the operation parameters of the instrument 40 sensors in order to fit them according to agrees. The research and development centre 47 also includes a user graphical interface 80 connected to means of programming 81 from where the parameters of operation of different instruments 40 from the plant can be reprogrammed.

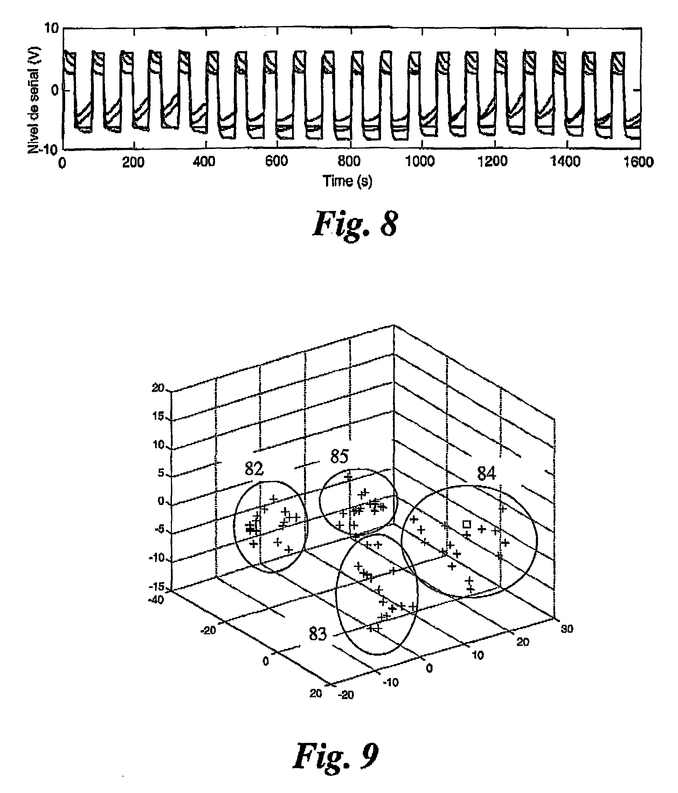

Figs. 7 and 8 are respectively examples of output signal level versus time response in front of vinegar of four metallic oxide sensors responses that are allocated inside the analysis chamber according to the present invention. Each curve corresponds to one of the four sensors. In Fig. 7, the sensors are excited according to a sawtooth wave that includes four cycles: a first cycle of chamber cleaning in which a cleaning fluid is circulated; a second cycle of reference in which a reference fluid is circulated; a cycle of analysis of the sample in which the sample of the fluid to analyse is circulated; and a final purge or drain cycle. In each cycle, a modulation of the excitation in the form of a single sawtooth takes place. In fig. 8 the modulation is in pulse waveform and such includes the same cycles but with five pulsations in each cycle.

In Fig. 9, a three-dimensional graph showing an olfactory map for four substances: coffee 82; cedar wood 83; tobacco 84; and air 85, as a result of the respective sample analysis, is shown. It must be noted that the three-dimensional axes system responds to complex data processing calculus and it is not representative of physical spatial coordinates. It can be observed how identifying points for each substance form concentrated groups in different regions of the shown three-dimensional space.

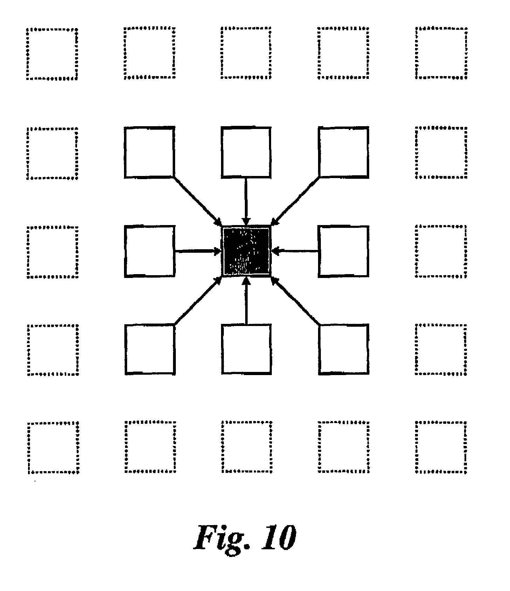

Finally, in Fig. 10 a system for analysis, identification and quantification of gases and liquids is diagrammatically represented. The system comprises a series of instruments like those herein described being linked in a logic form over a communications network so that the operation of each of said analysis, identification and quantification instruments is able to be supplemented with information received from one or more of the other instruments in said system. In the illustrative drawing of an instrument network, the unit stood out by a dark gray tone performs its analysis using to itself information from the other units (those that are closer, in this case the neighbouring ones).

## Claims

1. Instrument for analysis, identification and quantification of gases or liquids comprising:
a housing;
at least one sensor module including an analysis chamber (5) that lodges at least one chemical sensor (14);
at least two inlet ports (1a, 1b, 1c) and associated means (3a, 3b, 3c, 7) for selectively introducing in a controlled way two or more gas or liquid samples to the analysis chamber (5) and for circulating these samples therethrough, constituting a programmable flow subsystem;
a unit (17) for processing the data collected by said at least one sensor (14) and for the control of the operation of said means (3a, 3b, 3c, 7) for introducing and circulating the of gas or liquid samples;
**characterised in that** it further includes peripheral modules (19,20,21) comprising at least:
- one feeding module (19);
- a sensor signal acquisition and excitation module (20) that controls the operation of said at least one sensor (14) according with an excitation wave parametrically defined and adaptable; and
- a power module (21) to operate said means(3a, 3b, 3c, 7),
said peripheral modules (19, 20, 21) being associated to said processing unit (17) and applied jointly therewith to an active control of the operation of said at least one chemical sensor (14) providing an excitation waveform for this sensor (14) adapted for each specific application, based on the characteristics of the sample(s) composition to analyse.

2. Instrument, according to claim 1, **characterised in that** the sensor module includes a set of four or more metallic oxide sensors and a temperature sensor associated to this metallic oxide sensors set, which are located inside the analysis chamber enabling the acquisition of data from gas samples.

3. Instrument, according to claim 1, **characterised in that** the sensor module includes one or more non-selective metallic working electrodes, a reference electrode and a counter electrode, altogether being located inside the analysis chamber and governed by a potenciostat enabling the acquisition of data from liquid samples,

4. Instrument, according to claim 1, **characterised in that** it comprises several sensor modules connected in series to make sequentially circulate therethrough a sample or samples to be analysed, a chemical filter being arranged between each pair of said sensor modules and prepared to retire/absorb determined one or more components of the sample.

5. Instrument, according to claim 4, **characterised in that** said chemical filters are of different types and are arranged in a predetermined order so that they absorb sequentially different substances of the samples in said predetermined order.

6. Instrument, according to claim 1, **characterised in that** it comprises a manifold or collector (2) to which said inlet ports (1a, 1b, 1c) are coupled, each inlet port being associated to a electrically controlled valve controlled in a programmable way for said processing unit (17).

7. Instrument, according to claim 1, **characterised in that** said processing unit (17) is an embedded computer (18) connected to said peripheral modules (20, 21).

8. Instrument, according to claim 2, **characterised in that** it includes power limiting means associated to circuit means to activate heaters (25) associated to said sensors (14).

9. Instrument, according to claim 1, **characterised in that** one of said peripheral modules is a module prepared to be connected to and to reach communication networks in order to provide a control and/o remote programming, remote acquisition or analysis of the samples or recovery of previously acquired data, stored in a database.

10. Instrument, according to claim 9, **characterised in that** connection of said instrument with the communication network is performed through a serial port of the embedded computer.

11. Instrument, according to claim 9, **characterised in that** connection of said instrument with the communication network is performed through a network devoted device.

12. Instrument, according to claim 1, **characterised in that** the processing unit (17) includes an associated data base containing definition of the data acquisition procedures from the samples, such as control of the means to circulate the samples and control of the excitation waveform of the sensors

13. Instrument, according to claim 1, **characterised in that** it is constituted in a portable unit.

14. Instrument, according to claim 1, **characterised in that** all the configuration and operation of the instrument is contained in a database comprising:
- definition of the sample treatment procedure;
- definition of the sample patterns;
- class database;
- database containing component concentrations in the treated or to be treated samples; and
- definition of the processing of the signals.

15. System for the analysis, identification and quantification of gases and liquids that comprises several instruments according to one of claims 1 to 15, logically connected among them through a communication network so that the operation of each of said instruments can be supplemented with information received from one or more of the remaining instruments of said system.

16. Method for the analysis, identification and quantification of gases and liquids by using an instrument comprising:
at least a sensor module including an analysis chamber (5) that lodges at least one chemical sensor (14);
at least two inlet ports (1a, 1b, 1c) and associated means (3a, 3b, 3c, 7) for selectively introducing in a controlled way two or more gas or liquid samples to the analysis chamber (5) and for circulating these samples therethrough, constituting a programmable flow subsystem;
a unit (17) for processing the data collected by said at least one sensor (14) and for the control of the operation of said means (3a, 3b, 3c, 7) for introducing and circulating the of gas or liquid samples; and
peripheral modules (19, 20, 21) associated to said processing unit (17),
**characterised in that** the operation of said at least one sensor (14) is performed by exciting said sensor according with an excitation wave parametrically defined and adaptable, applied in a way that allow to be modified in successive steps in order to reach an optimal response.

17. Method, according to claim 16,**characterised in that** it comprises following steps:
a) selecting the programmable flow subsystem input of interest;
b) circulating the sample through the sensor chamber;
c) acquiring a response from the sensor to one form of an excitation wave selected during a programmed period of time;
d) processing said signal response to form a pattern;
e) comparing each new individual patter with a set of patterns previously stored in a database in order to produce an optimised response in the form of a tag or linguistic term.

18. Method, according to claim 16, **characterised in that** it comprises following steps:
a) selecting the programmable flow subsystem input of interest;
b) circulating the sample through the sensor chamber;
c) acquiring a response from the sensor to one form of an excitation wave selected during a programmed period of time;
d) processing said response forming a pattern; and
e) comparing each new individual patter with a set of patterns previously stored in a database in order to produce an optimised response in the form of a concentration value of at least one of the components of the sample.

19. The method according to one of the claims 17 or 18, **characterised in that** all the set of patterns are stored in a database.

20. Method to optimise the operation of an instrument according to claim 1, **characterised in that** it comprises following steps:
a) defining a quality factor;
b) defining a parametrical excitation waveform;
c) acquiring and processing the responses of the said at least one sensor (14), according to said current excitation waveform in order to form a pattern;
d) evaluating said quality factor with regard to a set of previously calculated patterns;
e) changing the parameter of the current excitation waveform in such a way that the quality factor is improved; and
f) iterating the steps c) to e) until a convergence criteria is attained.

21. The method of the claim 20 wherein the quality factor is an average distance calculated between groups of pattern data corresponding to the same kind of sample.

22. The method of claim 20, wherein the quality factor is the regression error.

23. The method of claim 20, wherein one of the parameters includes the activation or deactivation of one or more of the sensors (14).

24. A method for remote connection between a client and a server through a control architecture of an instrument according to claim 1, **characterised in that** it comprises following steps:
a) proceeding to an identification process;
b) performing a petition for a control operation or reprogramming;
c) generating a corresponding response from the instrument.

25. The method of claim 24, wherein the petition for a control comprises:
a) acquiring the value of a given set of sensors;
b) controlling some operation parameters such as sensor's modulation temperature and flow subsystem control conditions;
c) control of the system procedures including:
i) one order to start a sampling;
ii) one order to start a learning step;
iii) one order to classifying; and
iv) one order for a sample quantification analysis; and
d) temporisation of instructions in section c).

26. The method of claim 24 wherein the reprogramming petition comprises:
a) modifying the available sample treating procedure;
b) modifying the available signal processing procedures;
c) modifying the available active control excitation forms; and
d) modifying the existing databases.

27. A method for a remote activation of an instrument according to claim 1, **characterised in that** it comprises following steps:
a) perform an analysis of a sample according to one of the methods described in claims 17 or 18; and
b) send an informative signal to said instrument, internal or external, indicating that a defined event occurred.
